# EUROPEAN PATENT APPLICATION

(11) **EP 4 461 740 A1**
(43) Date of publication of application: **13.11.2024**
(21) Application number: 23177927.3
(22) Date of filing: 07.06.2023
(51) Int. Cl.: C07K 14/395, C12G 3/025, C12N 1/18, C12R 1/865

(54) **A MODIFIED YEAST STRAIN FOR LOW ALCOHOL FERMENTATION**

(30) Priority: 12.05.2023 EP 23173167
(71) Applicant: BRAIN Biotech AG, 64673 Zwingenberg (DE)
(72) Inventor: The designation of the inventor has not yet been filed
(74) Representative: Fabry, Bernd

(57) **Abstract**

The present invention discloses recombinant yeast strains with reduced ethanol production during fermentation. Said strains are obtainable by introduction of a mutated SPT-15 gene of *S*. *cerevisiae* into the genome off the host strain. Further provided are methods for generation of said strain and uses thereof.

## Description

### FIELD OF THE INVENTION

The present invention relates to recombinant yeast strains with reduced ethanol production during fermentation genetically modified such that they express a recombinant SPT-15 gene of *Saccharomyces cerevisiae* origin. Such strains are of particular interest in the production of ethanol-reduced or ethanol-free food or beverage compositions. The present invention also relates to a process for obtaining these yeasts and to industrial uses thereof.

### BACKGROUND OF THE INVENTION

Yeast-dependent fermentation of beverage and food compositions such as beer, wine, and bread is commonly used to change the chemical identity of ingredients and alter properties of said compositions. For instance, brewer's yeast (*Saccharomyces cerevisiae*) is most commonly used in beer production for its ability to convert carbohydrates such as sugars into ethanol. *S*. *cerevisiae* has been cultivated by humans for thousands of years. Accordingly, traditional crossing and selection has generated a great variety of *S*. *cerevisiae* strains with desired properties such as characteristic flavor profiles, increased alcohol production or reduction of bitterness achieved during fermentation.

The selection of *S. cerevisiae* with increased ethanol tolerance to allows higher percentage brewing procedures generating high alcohol-content in beverages or the production of bioethanol useful in the fuel industry has been widely adapted by the industry. However, most recently there has been an increasing industrial demand for fermentation products such as beverages and food stuff with reduced or alcohol-free content due to, for instance, health concerns attributed to alcohol consumption. The production of alcohol can be prevented by omitting the addition of yeast cultures during fermentation of beverages and food stuff. However, yeasts not only produce alcohol but have a wide variety of benefits for beverage and food production. For instance, yeasts convert simple ingredients such as carbohydrates into complex compounds contributing to distinct sensatory properties of the product, which is highly desirable and expected by the consumer. Additionally, fermentation can lower concentrations of toxic, harmful, or undesired compounds found in beverage and food stuff thereby contributing to increased health benefits of fermented products and benefiting consumers more generally by providing multiple compounds associated with good health.

Accordingly, there is a high demand for yeasts with reduced ethanol production. In order to reduce alcohol production, multiple studies have addressed the regulation of ethanol metabolism in yeasts. The deletion of enzymes involved in ethanol production such as alcohol dehydrogenase 1 (ADH1) in *S. cerevisiae,* for instance, showed reduced ethanol production (Ida,Y., Journal of bioscience and bioengineering, 113(2), 192-195. 2012). However, while ADH1 mutants produced lower concentrations of ethanol initially, ethanol production increased in subsequent generations. Recovery of ethanol production in the ADH1 deletion strain is hypothesized to be a result of the upregulation of ADH isozymes occurring during long-term culturing. Stable reduction of ethanol production required the deletion of all six ADH isozymes. However, the simultaneous knockout of all ADH is technically challenging and associated with a decreased fitness of generated yeast strains.

Engineering yeast strains with the capacity of redirecting carbon away from ethanol production to other endpoints is an effective approach for ethanol reduction during fermentation. Increasing the production of glycerol, for instance, has been highly effective in reducing ethanol production (Otterstedt, K. EMBO reports, 5: 532-537 2004). However, high concentrations of by-products, such as acetate, acetaldehyde, and acetoin, can accumulate affecting sensatory properties of products and health benefits of products.

Further research has elucidated the impact of the transcription factor TATA-binding Protein (TBP) encoded by the SPT-15 gene on ethanol resistance and production in yeast strains. This protein is one of the main DNA binding proteins that regulate promoter specificity in yeast. Mutated SPT-15 has been associated with increased ethanol resistance (US8809060B2). Most recently, a SPT-15 variant has been engineered that reduces ethanol production in *S. cerevisiae* yeast strains by over 30% compared to a control strain, which have been transformed with a plasmid encoding said SPT-15 mutant gene under the transcriptional control of the common TEF1 promoter (Du et al., Front. Microbiol., 2020).

While progress has been made in providing low-ethanol producing yeast strains, several challenges remain. The reduction of ethanol production by provided yeasts often involved negative changes of other fermentation properties such as growth and flavor profiles affected by by-products, insufficient reduction of ethanol production, and relapse of ethanol production in subsequent generations.

The objective of the present invention was, therefore, to provide novel yeast strains characterized in that they produce reduced amounts of ethanol with little changes in other fermentation properties such as growth and flavor profile and allow stable and reproducible fermentation without relapse of ethanol production.

### SUMMARY OF THE INVENTION

A first aspect of the invention relates to a low-ethanol producing, recombinant yeast strain characterized in that it expresses a mutated SPT-15 gene, wherein the mutated SPT-15 gene encodes an amino acid sequence comprising at least three, more preferred four, and most preferred five mutations at positions selected from the group of I46, D56, S118, Y195, and L205 in the amino acid sequence of the wild-type *Saccharomyces cerevisiae* SPT-15 gene and the strain comprises at least one genomic copy of said mutated SPT-15 gene.

In a preferred embodiment, said yeast strain is characterized in that it expresses a mutated SPT-15 gene, wherein the mutated SPT-15 gene encodes an amino acid sequence comprising mutations at positions I46, D56, S118, Y195, and L205 in the amino acid sequence of the wild-type *Saccharomyces cerevisiae* SPT-15 gene and the strain comprises at least one genomic copy of said mutated SPT-15 gene. In a preferred embodiment, said mutations at position I46, D56, S118, Y195, and L205 are I46M, D56G, S118P, Y195H, and L205S respectively. In some embodiments the mutated SPT-15 gene encodes an amino acid sequence according to SEQ ID NO 1. In some embodiments the mutated SPT-15 gene comprises nucleic acid sequence according to SEQ ID NO 2.

In some embodiments said at least one copy of the mutated SPT-15 gene is inserted into the genomic region selected from the group of intergenic regions, regulatory regions, genic regions, or combinations thereof. In a preferred embodiment, said at least one copy of the mutated SPT-15 gene is inserted into a genic region of the yeast strain according to the invention.

The recombinant yeast strains according to the invention may be of any yeast genus known. In a preferred embodiment, the recombinant yeast strain according to the invention is of genus selected from the group of *Saccharomyces spp, Kazachstania spp, Torulaspora spp,* and *Pichia spp.* In a more preferred embodiment, said recombinant yeast strain is of genus *S. cerevisiae* or *S*. *pastorianus,* most preferred of genus *S. cerevisiae.*

In some aspects, the recombinant yeast strain according to the invention is further characterized in that it expresses a wild-type SPT-15 gene from its genomic locus at levels comparable with wild-type *S. cerevisiae* strains. In some embodiment, said yeast strain is characterized in that it is deficient in at least one gene associated with ethanol metabolism selected from the group of genes encoding alcohol dehydrogenases, aldehyde dehydrogenases, triose-phosphate isomerases. In a preferred embodiment, said yeast strain is deficient in at least one gene encoding an alcohol dehydrogenase. In a more preferred embodiment, said yeast strain is further characterized in that it is deficient in at least one gene selected from the group of ADH1, ADH2, ADH3, ADH4, ADH5, SFA1, ALD6, PDC2, TPI1 or combinations thereof, most preferably deficient in ADH-1.

In some aspects, the at least one copy of the mutant SPT-15 gene is inserted into the genomic ADH-1 gene locus of the recombinant yeast strain according to the invention in a way that said insertion functionally disrupts the ORF of said ADH-1 gene and co-opts its regulatory regions for its expression.

In a preferred embodiment, the recombinant yeast strain according to the invention is *Saccharomyces cerevisiae* strain BR 23007 deposited under DSM 34577 on 5^{th} of April 2023 at the Institute DSMZ.

In some aspects, the recombinant yeast strain according to the invention is further characterized in that it produces about 10% to about 90% less ethanol compared to a wild-type *Saccharomyces cerevisiae* strain.

In another aspect, disclosed is a low-ethanol producing, recombinant yeast strain according to *Saccharomyces cerevisiae* strain BR 23007 deposited under DSM 34577 on 5^{th} of April 2023 at the Institute DSMZ. In some embodiments, said recombinant yeast strain is further characterized in that
- the precursor yeast strain is of genus *S. cerevisiae,*
- the mutant SPT-15 gene comprising nucleic acid sequence according to SEQ ID NO 02 is inserted into the ORF of the ADH-1 locus of the precursor strain, and
- it produces at least 30% less ethanol compared to a wild-type *S. cerevisiae* yeast strain.

Further disclosed is a method for the generation of a recombinant yeast strain according to any of the preceding claims comprising, consisting, or essentially consisting of the following steps:
(a) providing a precursor yeast strain, at least one polynucleotide encoding said mutant SPT-15 gene and selecting at least one insertion locus comprising the genome of said strain,
(b) inserting said at least one polynucleotide into said at least one insertion locus of the precursor yeast strain thus generating yeast mutant strains,
(c) optionally isolating single colonies of the yeast mutant strains thus obtained,
(d) selecting one or more of said yeast mutant strains, which produce ethanol at a lower rate than the precursor yeast strain.

In some embodiments, the recombinant yeast strain according to *Saccharomyces cerevisiae* strain BR 23007 deposited under DSM 34577 is obtainable or obtained according to the method of the invention.

Further disclosed is the use of the recombinant yeast strain according to the invention for fermentation.

A further aspect relates to the use of the recombinant yeast strain according to the invention for the preparation of alcohol-reduced foods and beverages

### DEFINITIONS

For convenience, certain terms employed in the specification, examples, and appended claims are collected here.

As used herein, the singular form "a," "an," and "the" include plural references unless the context clearly dictates otherwise. For example, use of the term "a yeast cell" or "yeast" optionally includes, as a practical matter a plurality of yeast cells or yeasts.

As used herein, the term "about" indicates that a value includes the inherent variation of error for the method being employed to determine a value, or the variation that exists among.

The expression "yeast strain" denotes a relatively homogeneous population of yeast cells. A yeast strain is obtained from a clone, a clone being a population of yeast cells that is obtained from a single yeast cell.

A "chimeric gene" or "chimeric construct" as used herein is a recombinant nucleic acid sequence in which a promoter or regulatory nucleic acid sequence is operably linked to, or associated with, a nucleic acid sequence that codes for a mRNA encoding an amino acid sequence, or other RNAs such as gRNAs, such that the regulatory nucleic acid sequence is able to regulate transcription or expression of the associated nucleic acid sequence. The regulatory nucleic acid sequence of the chimeric gene is not operably linked to the associated nucleic acid sequence as found in nature.

As used herein, the term "identity" when used in relation to nucleic acids, describes the degree of similarity between two or more nucleotide sequences. The percentage of "sequence identity" between two sequences can be determined by comparing two optimally aligned sequences over a comparison window, such that the portion of the sequence in the comparison window may comprise additions or deletions (gaps) as compared to the reference sequence (which does not comprise additions or deletions) for optimal alignment of the two sequences. The percentage is calculated by determining the number of positions at which the identical 10 nucleic acid base or amino acid residue occurs in both sequences to yield the number of matched positions, dividing the number of matched positions by the total number of positions in the window of comparison, and multiplying the result by 100 to yield the percentage of sequence identity. A sequence that is identical at every position in comparison to a reference sequence is said to be identical to the reference sequence and vice-versa. An alignment of two or more sequences may be performed using any suitable computer program. For example, a widely used and accepted computer program for performing sequence alignments is CLUSTALW vl .6 (Thompson, et al. (1994) Nucl. Acids Res., 22: 4673-4680).

### DETAILED DESCRIPTION OF THE INVENTION

In order to obtain a recombinant yeast strain that produces lower levels of ethanol compared to an unmodified wild-type/control strain and retaining desirable fermentation properties, a yeast strain has been genetically modified so as to express at least one copy of a mutated SPT-15 from a genomic locus of said yeast strain.

Thus, a first object of the present invention refers to a low-ethanol producing yeast strain characterized in that it expresses a mutated SPT-15 gene, or a homologue or paralogue thereof, comprising its genome.

In particular, an object of the present invention refers to a low-ethanol producing recombinant yeast strain characterized in that it expresses a mutated SPT-15 gene, wherein the mutated SPT-15 gene encodes an amino acid sequence comprising at least three, more preferred four, and most preferred five mutations at positions selected from the group of I46, D56, S118, Y195, and L205 in the amino acid sequence of the wild-type *Saccharomyces cerevisiae* SPT15 gene and wherein the strain comprises at least one genomic copy of said mutated SPT-15 gene.

The key transcription factor TATA-binding Protein (TBP) encoded by the wild-type *Saccharomyces cerevisiae* SPT-15 gene influences the transcription of a multitude of genes in yeasts and has been implicated in ethanol production. It has been surprisingly found that the expression of at least one genomic copy of the mutated SPT-15 gene of wild-type *Saccharomyces cerevisiae* according to the invention stably reduces the ethanol production of said yeast strain during fermentation for multiple generations.

### YEAST STRAIN

A low-ethanol producing, recombinant yeast strain (hereinafter recombinant yeast strain) according to the present invention or the unmodified precursor (parent) yeast strain may be of any yeast genus known. In some embodiments, the yeast strain is suitable for beverage, food, and/or feed fermentation, which can safely be used in the fermentative production of beverage, food and/or feed without causing health risk for the consumer of said beverage, food and/or feed. In some embodiments, the recombinant yeast strain according to the invention is selected from the genus *Saccharomyces.*

A precursor yeast strain suitable for modification according to the invention may be any yeast strain not genetically modified according to the invention. The skilled person in the art is aware of multiple selection methods to select a precursor yeast strain suitable for modification based on desired fermentation properties and applications prior to modification. In some embodiments, the precursor yeast strain is selected for desired fermentation properties based on individual applications such as food and beverage production. In some embodiments the precursor yeast strain is a wild-type *S. cerevisiae* strain.

In a preferred embodiment, the recombinant yeast strain according to the invention is of type *Saccharomyces cerevisiae.* In a more preferred embodiment, the recombinant yeast strain according to the invention is *Saccharomyces cerevisiae* strain BR 23007 deposited under DSM 34577 on 5^{th} of April 2023 at the Institute Deutsche Sammlung von Mikroorganismen und Zellkulturen GmbH (hereinafter DSMZ).

### MUTATED SPT-15 GENE

The introduction of targeted mutations in the SPT-15 gene of wild-type *Saccharomyces cerevisiae* and subsequent transformation has been previously demonstrated to decrease ethanol production of *Saccharomyces cerevisiae* (Du et al., Front. Microbiol., 2020). Surprisingly it has been found that integration of said mutated SPT-15 gene into the genome of a yeast strain according to the invention reduces ethanol production during fermentation compared to a control strain lacking said mutated SPT-15 gene.

The coding nucleic acid sequence (mRNA sequence) of the wild-type SPT-15 gene of *Saccharomyces cerevisiae* is disclosed in SEQ ID NO 08. The amino acid sequence encoded by said wild-type SPT-15 gene is disclosed in SEQ ID NO 09.

In some embodiments the mutated SPT-15 gene according to the invention encodes an amino acid sequence comprising mutations at positions I46, D56, S118, Y195, and L205 in the amino acid sequence encoded by the wild-type *Saccharomyces cerevisiae* SPT-15 gene. In a preferred embodiment, said mutations at position I46, D56, S118, Y195, and L205 are I46M, D56G, S118P, Y195H, and L205S respectively, which corresponds to amino acid sequence according to SEQ ID NO 1. In a more preferred embodiment, the mutated SPT-15 gene encodes an amino acid sequence with at least 10%, 20%, 30%, 40%, 50%, 60%, 70%, 80%, 90%, 95%, or 99% identity to amino acid sequence according to SEQ ID NO 01. In a more preferred embodiment, the mutated SPT-15 gene according to the invention encodes an amino acid sequence according to SEQ ID NO 01.

In some embodiments, the mutated SPT-15 gene according to the invention consists or comprises a nucleic acid sequence encoding the mutated SPT-15 gene according to the invention. The sequence of the mutated SPT-15 gene according to the invention may be adapted for its expression in the host yeast. Indeed, the person skilled in the art knows the notion of codon usage bias and how to adapt nucleic sequences for a particular codon usage bias without modifying the deduced protein.

SEQ ID NO 02 corresponds to a nucleic acid sequence encoding the amino acid sequence according to SEQ ID NO 1. In some embodiments, the mutated SPT-15 gene according to the invention consists or comprises a nucleic acid sequence with at least 10%, 20%, 30%, 40%, 50%, 60%, 70%, 80%, 90%, 95%, or 99% identity to nucleic acid sequence according to SEQ ID NO 02. In another embodiment, the mutated SPT-15 gene according to the invention consists or comprises nucleic acid sequence according to SEQ ID NO 02.

In some embodiments, the mutated SPT-15 gene according to the invention can be functionally linked to a promoter, a terminator or any other sequence required for its expression in yeast known by the skilled person in the art. In some embodiments, said mutated SPT-15 gene is functionally linked to a TEF1 promoter.

### GENOME INTEGRATION

Transformation of plasmid DNA encoding recombinant proteins is most often used to temporarily induce expression of respective transgenes. However, plasmid DNA does not regularly integrate stably into the host genome and can be lost or diluted relatively fast in subsequent generations. While plasmid DNA may integrate at random integration sites/loci within the host genome, expression may be lost or prevented by induction of heterochromatin at insertion sites. Additionally, random integration may disrupt significant regulatory and/or genic regions leading to decreased fitness overall and further undesirable defects. This is particularly challenging for the food and beverage industry, which relies on rigorously tested and characterized yeast strains for fermentation that can deliver a consistent and safe product.

Surprisingly, the targeted introduction of the mutated SPT-15 gene into the genome of the host strain allows stable expression of the recombinant protein in subsequent generations. Accordingly, in some embodiments, the genome of the recombinant yeast strain according to the invention comprises at least one copy of the mutated SPT-15 gene.

The selection of the insertion locus or loci (= target locus/loci for the mutated SPT-15 gene) are critical for expression of the encoded transgene and production of the encoded amino acid sequence/oligopeptide. Expression of a transgene requires regulatory elements, for instance, driving polymerases recruitment and expression. The selection of said regulatory elements is critical to titrate expression levels of the transgene. In some embodiments, the mutated SPT-15 gene is inserted into the genomic region or regions (insertion locus/loci) of the precursor (parent) yeast strain selected from the group of intergenic regions, regulatory regions, genic regions, or combinations thereof.

Integration of a transgene into the open reading frame (ORF) of an endogenous gene of the host cell may allow co-option of regulator elements driving expression of the targeted gene. Simultaneously, insertion of the transgene most often result in the disruption of the endogenous target gene and reduction or abolition of target protein production. Accordingly, the selection of the target region of insertion (insertion locus) of the transgene can have detrimental impact on the host cell in regards to growth, fermentation properties and ethanol production.

Surprisingly, it has been discovered that integration of the mutant SPT-15 gene according to the invention into genes associated with ethanol metabolism (=target genes) can reduce ethanol production of the generated recombinant yeast strain even further compared to sole expression of the mutated SPT-15 gene in yeast strains. Accordingly, in some embodiments, the mutant SPT-15 gene according to the invention is inserted into the ORF of a gene associated with ethanol metabolism selected from the group of alcohol dehydrogenases, aldehyde dehydrogenases, triose-phosphate isomerases. In a preferred embodiment, the mutant SPT-15 gene according to the invention is inserted into the ORF of at least one gene selected from the group of ADH1, ADH2, ADH3, ADH4, ADH5, SFA1, ALD6, PDC2 and TPI1.

Expression of the mutated SPT-15 gene according to the invention requires regulatory elements such as promoter. Introduction of said SPT-15 gene into a genomic locus such as the ORF of a target gene may coopt the host's endogenous regulatory elements. However, in some instances, expression may be less efficient due to, for instance, promoter strength of the targeted gene. Accordingly, in some embodiments the ORF and regulatory elements of at least one target gene maybe be replaced by the mutated SPT-15 gene according to the invention functionally linked to regulatory elements such as promoters of desired strength. In some embodiment the mutated SPT-15 gene according to the invention is functionally linked to a promoter and replaces the ORF and promoter of the at least one target gene. In some embodiments, the at least one target gene is selected from the group of ADH1, ADH2, ADH3, ADH4, ADH5, SFA1, ALD6, PDC2 and TPI1.

Surprisingly, targeted introduction of the mutant SPT-15 gene into the ORF of alcohol dehydrogenase 1 (ADH-1) of yeast strains of, for instance, *S. cerevisiae* has been shown to be especially useful for stably lowering ethanol production in the recombinant yeast strain according to the invention compared to unmodified yeast strains. This is especially surprising since it is well established that knockout or knockdown of ADH-1 in *S. cerevisiae* is initially associated with reduced ethanol production of affected yeast strains. However, ethanol production recovers to near control levels in subsequent generations, probably due to epigenetic adaptations such as increased expression of other alcohol dehydrogenase variants, e.g., ADH-2 in response to ADH-1 disturbance (Ida, Y., Journal of bioscience and bioengineering, 113(2), 192-195. 2012). Surprisingly, the combination of expression of the mutant SPT-15 gene according to the invention under control of the ADH-1 associated regulatory elements and the disruption of the ADH-1 gene results in a synergistical impact on ethanol production, wherein ethanol production is significantly reduced compared to a control yeast strain lacking expression of mutant SPT-15 and/or ADH-1 knockout.

While not wishing to be bound by any theory, we theorize that disruption of the ADH-1 locus by the targeted integration of the mutated SPT-15 gene according to the invention prevents expression of the disrupted ADH-1 gene thereby lowering ethanol production, while simultaneously co-opting the regulatory elements of the ADH-1 locus for expression of the mutated SPT-15 gene at levels ideal to synergistically lower ethanol production even further. The introduction of the mutated SPT-15 may disrupt regulatory networks usually involved in epigenetic adaptation of ethanol production in yeast strains deficient only in ADH-1 responsible for an increased expression of alcohol dehydrogenases variants, which commonly compensate lack of ADH-1 in subsequent generations. This unexpected result has multiple advantages over known methods for reduction of ethanol production during fermentation using yeasts such as multiple knockouts of all known alcohol dehydrogenase. Expression of the mutated SPT-15 gene and disruption of the ADH-1 gene is relatively well tolerated in recombinant yeast strains according to the invention with little to no impact on growth or other desirable fermentation properties. In stark contrast, the deletion of all alcohol dehydrogenases from the yeast genome represents a large genomic burden and may impact more generally growth and fermentation properties.

While integration of the mutated SPT-15 gene into the coding region of a gene involved in ethanol production is sufficient to drive expression of said mutated SPT-15 gene and simultaneously co-opt the regulatory elements of said gene involved in ethanol production driving expression of the transgene, similar results can be achieved by selecting or preparing precursor yeast strains deficient in said genes involved in ethanol production. Accordingly, in some embodiments, the recombinant yeast strain is further characterized in that it is deficient in at least one gene involved in ethanol production.

In some embodiments said strain is characterized in that it is deficient in at least one gene encoding an alcohol dehydrogenase, preferably deficient in at least one gene selected from the group of ADH1, ADH2, ADH3, ADH4, ADH5, SFA1 or combinations thereof and most preferably deficient in ADH1.

In some embodiments said strain is characterized in that it is deficient in at least one gene encoding an aldehyde dehydrogenase, preferably ALD6. In some embodiments said strain is characterized in that the ALD6 ORF and promoter is replaced by the mutated SPT-15 gene according to the invention functionally linked to a TEF1 promoter.

### ETHANOL PRODUCTION

The recombinant yeast strain according to the invention is characterized in that it has reduced ethanol production compared to the precursor strain used to generate the recombinant yeast strain according to the invention. Accordingly, the recombinant yeast strain according to the invention is characterized in that it produces about 5% to about 100% less ethanol, preferably about 10% to about 90%, preferably about 20% to about 80%, preferably about 30% to about 70%, more preferably about 35% top about 60%, and most preferably about 40% to about 60% less ethanol compared to an unmodified precursor or wild type yeast strain (control strain).

### STRAIN GENERATION

In one aspect, provided is a method for the generation of a recombinant yeast strain according to the invention comprising, consisting, or essentially consisting of the following steps:
(a) providing a precursor yeast strain, at least one polynucleotide encoding the mutant SPT-15 gene according to the invention and selecting at least one insertion locus comprising the genome of said strain,
(b) inserting said at least one polynucleotide into said at least one insertion locus of the precursor yeast strain thus generating yeast mutant strains,
(c) optionally isolating single colonies of the yeast mutant strains thus obtained,
(d) selecting one or more of said yeast mutant strains, which produce ethanol at a lower rate than the precursor yeast strain.

Targeted introduction/ insertion of genetic material into the genome of organisms has been greatly improved in recent times. In particular, CRISPR/Cas-dependent methods are well known to the person skilled in the art. The very efficient CRISPR/Cas technology, which has also been used in the Examples of this application, allows the guided introduction of distinct polynucleotide sequences at precise insertion loci of the target genome. It is based on the bacterial "immune system"-derived CRISPR (clustered regularly interspaced palindromic repeats) pathway and has been modified to edit basically any genome. By delivering the Cas nuclease (in many cases Cas9) complexed with a synthetic guide RNA (gRNA or sgRNA) in a cell, the cell's genome can be cut at a desired location depending on the sequence of the gRNA, allowing existing genes to be removed and/or insertion of new genes (e.g. DiCarlo et al 2013 Nucl Acids Res 41:4336-4343; Sander & Joung 2014 Nat Biotech 32:347-355).

One aspect of the invention refers to a recombinant yeast strain in which the mutated SPT-15 gene according to the invention has been inserted into the yeast strain's genome by using nuclease technology, more particularly by means of CRISPR/Cas technology.

In order to generate a recombinant yeast strain according to the invention, at least one synthetic guide RNA targeting the at least one insertion locus is expressed in the unmodified precursor yeast strain. Therefore, the application further provides at least one chimeric gene construct comprising a promoter which is active in a eukaryotic cell, more particularly in a yeast cell, operably linked to a CRISPR guide RNA targeting the insertion locus or loci. In some embodiments, said constructs comprises multiple gRNAs operably linked targeting distinct insertion loci. "Targeting" as used herein is equivalent to "annealing" or "binding" to the insertion locus. As a result, the CRISPR gRNA is complementary to a part of the insertion locus. In one embodiment, said promoter of said chimeric gene construct is also operably linked to a nucleic acid molecule encoding a Cas endonuclease, more particularly a BMC (BRAIN Metagenome Cas) endonuclease (WO 2022/184765 A1). In another embodiment, the chimeric gene comprising the targeting gRNA is expressed together with the chimeric gene comprising the Cas encoding nucleic acid molecule. In one embodiment, the chimeric gene constructs additionally comprise a 3' end region involved in transcription termination and/or polyadenylation.

Following Cas-mediated strand cleavage at the selected at least one insertion locus comprising the genome of the precursor yeast strain, the resulting cleavage products can be repaired by cellular mechanisms such as NHEJ. However, the provision of a donor constructs comprising the desired insertion sequence flanked by homology arms complementary to the cleaved strand ends allows the insertion of said sequence into at the insertion loci. Accordingly, further provided is at least one polynucleotide (donor construct) encoding the mutated SPT-15 gene according to the invention. In some embodiments, the polynucleotide comprising the mutated SPT-15 gene according to the invention further comprises homology arms able to hybridize with the cleavage strand ends of the insertion locus.

Said chimeric gene construct(s) and the donor construct(s) are expressed in yeast according to standard methods known to the skilled person allowing the insertion of the mutated SPT-15 gene according to the invention at the selected insertion loci within the genome of the recombinant yeast strain precursor.

Recombinant yeast strains are obtainable by the method of the invention. While the CRISPR/Cas technology approach is one particular example useful for the generation of the recombinant yeast strain according to the invention, it is acknowledged that multiple valid technologies exist able to engineer the genome of a recombinant yeast strain in a similar way such as the use of Transcription activator-like effector nucleases (TALENs), Zin-finger nucleases (ZFN) technologies and the likes known by the skilled person in the art.

Following the introduction of the mutated SPT-15 gene into the genome of the precursor yeast strain, the generated mutants are selected for lower production of ethanol. Ethanol production may be measured by methods known by the skilled person in the art such as determining ethanol content or concentration of fermentation medium comprising a yeast culture under fermentation conditions at defined end points such as 1 hour, 1 day, 3 days, 1 week, or 1 month. Said lower production is determined by comparing the ethanol production of the precursor yeast strain with the ethanol production of the mutants. In some embodiments, the ethanol production of the recombinant yeast strain according to the invention is about 0% to about 90%, more preferred about 0% to about 60%, more preferred about 0% to about 50%, more preferred about 10% to about 30%, more preferred about 20% to about 30% reduced compared to the precursor yeast strain as measured at same end point and under the same fermentation conditions. In some embodiment, the end point is 72h.

In some embodiments, the method according to the invention further comprises steps (e) to (f):
(e) culturing the one or more selected yeast mutant strains in a culture medium,
(f) recovering the one or more yeast mutants from the culture medium to provide a starter culture composition, and
(g) optionally, concentrating the one or more yeast mutants or starter culture composition.

### YEAST STRAIN DEPOSITED UNDER DSM 34577

A further aspect of the present invention refers to a low-ethanol producing, recombinant yeast strain according to *Saccharomyces cerevisiae* strain BR 23007 deposited under DSM 34577 on 5^{th} of April 2023 at the Institute DSMZ. Said strain was deposited under the provisions of the Budapest Treaty in the Leibniz Institute DSMZ - German Collection of Microorganisms and Cell Cultures. It has been assigned to the Accession number DSM 34577.

Said strain was obtained according to the method of the invention. A precursor yeast strain of genus *S. cerevisiae* was selected and the nucleic acid sequence according to SEQ ID NO 02 was integrated into the protein coding region of the ADH-1 locus. Said yeast strain has been selected by the Applicant as having reduced ethanol production properties compared to the precursor strain.

Said yeast strain is characterized in that
- the yeast strain is of genus *S. cerevisiae,*
- the mutant SPT-15 gene comprising nucleic acid sequence according to SEQ ID NO 02 is inserted into the ORF of the ADH-1 locus of the precursor strain, and
- it produces at least 30% less ethanol compared to a wild-type *S. cerevisiae* yeast strain.

In a preferred embodiment the recombinant yeast strain according to *Saccharomyces cerevisiae* strain BR 23007 deposited under DSM 34577 on 5^{th} of April 2023, belongs to risk group 1. The term "risk group" is a classification system which is used in many countries for the classification of microorganisms. The affiliation to a risk group depends for example on the following factors
- Pathogenicity of the organism
- Mode of transmission and host range
- Availability of effective preventive measures (e.g. vaccines)
- Availability of effective treatment (e.g. antibiotics)
- All microorganisms which belong to risk group 1 are not associated with disease in healthy adult humans (see NIH Guidelines in Recombinant DNA, April 2002).

In some embodiment the recombinant yeast strain according to *Saccharomyces cerevisiae* strain BR 23007 deposited under DSM 34577 on 5th of April 2023, deviates from wild-type *Saccharomyces cerevisiae* in the production of fermentative metabolites during juice matrix fermentation as follows:
- Ethanol production after 72 hours is at least 30% lower compared to wild-type *Saccharomyces cerevisiae*
- Glycerol production doubled compared to wild-type *Saccharomyces cerevisiae*

### INDUSTRIAL APPLICATION

The invention also relates to a yeast or yeasts obtained by culturing one of the recombinant yeast strains of the invention. Processes for culturing a yeast strain are known in the art, and those skilled in the art know how to optimize the culture conditions for each strain depending on its nature.

The yeasts obtained by the multiplication of the strains according to the invention have useful performances for industrial applications such as food-, feed-, and beverage production, biomass production, flavor production, the production of secondary metabolites, protein production, ethanol production, or the production of yeast extract. In other words, the yeasts obtained by culturing the strains according to the invention are efficient in the final industrial use for which they are intended.

The yeasts according to the invention are particularly useful in industrial applications such as the commercial fermentation of feed, food stuff, and beverages to produce an ethanol-reduced product compared to products fermented with wild-type/conventional yeast strains as known in the art.

The invention further relates to the use of the recombinant yeast strain according to the invention for fermentation.

Further disclosed is a method for production of the alcohol-reduced beverage according to the invention comprising, consisting, or essentially consisting of the following steps:
a) providing plant material and yeasts obtained by culturing at least one of the recombinant yeast strains of the invention,
b) contacting said plant material with said yeasts,
c) fermenting the mixture thus obtained,
d) and optionally separating the yeasts from the mixture to obtain the alcohol-reduced beverage.

Plant material according to the method of the invention may be any plant material useful for the production of a fermented beverage as known by the skilled person in the art. Non-limiting examples of plant materials are vegetables, vegetable juices, fruits, fruit juices, plant infusions such as teas, grains, and dairy or combinations thereof.

In a preferred embodiment of the method, said recombinant yest strain is yeast strain according to *Saccharomyces cerevisiae* strain BR 23007 deposited under DSM 34577.

A further aspect relates to an alcohol-reduced beverage obtainable or obtained by the method of the invention.

In some embodiments, said alcohol-reduced beverages are further characterized in that the ethanol content is reduced by at least 10%, 20%, 30%, 40%, 50%, 60%, 70%, 80%, 90% or 99% compared to beverages produced using the same fermentation method and conventional yeasts.

Unless otherwise defined, all the technical and scientific terms used in the specification have the same meaning as that commonly understood by one skilled in the field to which this invention belongs. Likewise, all the publications, patent applications, all the patents and any other references mentioned herein are incorporated by way of reference.

### BRIEF DESCRIPTION OF THE FIGURES

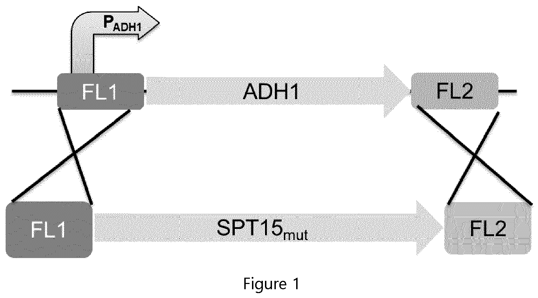

**Figure 1** shows a schematic representation of a knock-in of mutated SPT15 according to the invention into the ADH1 gene locus of Saccharomyces cerevisiae via homologous recombination by CRISPR engineering using BMC (as disclosed in WO 2022/184765 A1) according to the method of the invention.

### EXAMPLES

The examples below describe some embodiments of the present invention. However, it is understood that the examples and the figures are presented only by way of illustration and do not in any way limit the scope of the invention.

### Example 1

### Construction of the recombinant yeast strain according to Saccharomyces cerevisiae strain BR 23007 deposited under DSM 34577

A *Saccharomyces cerevisiae* ADH1:SPT15 knock-in mutant (yeast strain according to Saccharomyces cerevisiae strain BR 23007 deposited under DSM 34577) was established using a CRISPR/Cas vector system as described in WO 2022/184765 A1. A *S. cerevisiae* strain (HF13011) was used as precursor/progenitor strain.

### CRISPR/Cas12a vector system

The yeast cultivation, transformation procedure and DNA techniques were performed as described in WO 2022/017633 A2. The Cas12a nuclease used was BMC09 (SEQ ID NO 03). The chimeric guide RNA contained an ADH1 specific 23 bps spacer sequence (SEQ ID NO 04). The complete sequence of the CRISPR/BMC09 vector providing the nuclease is disclosed in SEQ ID NO 05. The complete sequence of the vector providing the guide RNA targeting ADH1 is provided in SEQ ID NO 06.

### Design of a homology directed repair template (HDR-template)

The 1760 bps ADH1-SPT15 repair template was designed to completely replace the ADH1 open reading frame (thereby abolishing the PAM and proto-spacer sequence) by homologous recombination and insert the mutated SPT15 (SEQ ID NO 02) which is concomitantly put under transcriptional control of the ADH1 promotor. Within the HDR-template, the mutated SPT15 sequence was flanked by 370 and 367 bps sequences homologous to the chromosomal ADH1 target region. The complete sequence of the ADH1 HDR-template is provided as SEQ ID NO 07.

### Phenotypic characterization of S. cerevisiae knock-in mutants

The ADH1 knock-in mutant DSM34577 was phenotypically characterized in a juice-based substrate. Yeast biomass was freshly prepared as follows. YPD agar plates were inoculated from cryogenic glycerol stocks by streaking and incubating at 28°C for 48 hours. Liquid YPD cultures (50ml in 500ml unbaffled shake flasks with silicose stoppers) were inoculated from single colonies and incubated with shaking for 24hours at 28°C.

Overview of the tested yeast strains

| Strain | Species | Genotype |
|---|---|---|
| HF13011* | S. cerevisiae | Wine yeast |
| DSM34577 | S. cerevisiae | derivative of HF13011 |
| HF13015* | T. delbrueckii | Wildtype wine yeast |

| | | |
|---|---|---|
| * commercially available at WeissBioTech GmbH (Ascheberg, Germany) | | |

The composition of the juice fermentation substrate was as follows:

| Ingredient | Final concentration |
|---|---|
| Apple Juice Concentrate 65 Brix (Vögele KG, Germany) | 2% (v/v) |
| Pear Juice Concentrate 60 Brix (Vögele KG, Germany) | 1.5% (v/v) |
| White Grape Juice Concentrate 65 Brix (Vögele KG, Germany) | 1.5% (v/v) |
| Green Tea Powder (Martin Bauer, Germany) | 0.5 g/L |
| Sucrose | 15 g/L |

Juice substrate was freshly prepared using tap water sterilized by autoclaving. Juice fermentation was performed in 300ml unbaffled shake flasks with silicose stoppers containing 50ml substrate each. The fermentation was performed at 28°C with shaking for 3 days. Prior to fermentation the juice fermentation cultures (J_1 to J_4) were inoculated with freshly prepared yeast cells as follows:

| Fermentation batch | Strain A | Strain A cfu/L | Strain B | Strain B cfu/L |
|---|---|---|---|---|
| J_1 | HF13011 | 5E+08 | - | - |
| J_2 | DSM34577 | 5E+08 | - | - |
| J_3 | HF13011 | 5E+08 | HF13015 | 1E+09 |
| J_4 | DSM34577 | 5E+08 | HF13015 | 1E+09 |

Baseline OD (600nm), pH and Brix values prior to fermentation were as follows:

| Fermentation batch | OD (600nm) | pH | Brix |
|---|---|---|---|
| J_1 | 0.13 | 4.12 | 6.2 |
| J_2 | 0.16 | 4.09 | 6.4 |
| J_3 | 0.34 | 4.07 | 6.5 |
| J_4 | 0.34 | 4.07 | 6.4 |

After fermentation, the fermented products were assessed with respect to OD (600nm), pH and Brix. Samples for HPLC and ethanol analytics were obtained by centrifugation, sterile filtration and stored at -20°C prior to use. The ethanol concentration was determined using a commercial kit (Enrytec Liquid Ethanol, R-Biopharm, Germany).
OD (600nm), pH, Brix and Ethanol values after 3-day fermentation were as follows:

| Fermentation batch | OD (600nm) | pH | Brix | Ethanol g/L |
|---|---|---|---|---|
| J_1 | 9.6 | 3.61 | 3.6 | 4.92 |
| J_2 | 10.0 | 3.65 | 3.9 | 2.75 |
| J_3 | 16.8 | 3.46 | 3.8 | 2.92 |
| J_4 | 18 | 3.49 | 3.7 | 1.89 |

The levels of glucose, fructose, and glycerol were measured by high performance liquid chromatography (HPLC).
- Instrumentation:
   - Agilent 1200 HPLC-DAD coupled to RI detector
   - BIORAD Aminex HPX-87H, 300x7,8 mm, 9 µm particles (anion exchange/ligand exchange chromatography)
- Mobile Phase:
   - Isocratic 30 mM H₂SO₄ + 1% AcN
   - Flow: 0.6 ml/min
- Injection volume 20 µl (MTP format)
- Column temperature: 60 °C
- Total run time: 25 min per sample

HPLC results for juice fermented products were as follows

| Fermentation batch | Glucose mM | Fructose mM | Glycerol mM |
|---|---|---|---|
| J_1 | 52.95 | 129.30 | 18.47 |
| J_2 | 65.56 | 111.14 | 38.32 |
| J_3 | 35.80 | 119.37 | 14.49 |
| J_4 | 33.08 | 108.00 | 17.91 |

As shown above, the use of the recombinant yeast strain according to *Saccharomyces cerevisiae* strain BR 23007 deposited under DSM 34577 showed significantly reduced ethanol production compared to the precursor yeast strain (44% reduction) while showing increased glycerol production (> 2-fold increase).

## Claims

1. A low-ethanol producing, recombinant yeast strain **characterized in that** it expresses a mutated SPT-15 gene,
wherein the mutated SPT-15 gene encodes an amino acid sequence comprising at least three mutations at positions selected from the group of I46, D56, S118, Y195, and L205 in the amino acid sequence of the wild-type *Saccharomyces cerevisiae* SPT-15 gene and
the strain comprises at least one genomic copy of said mutated SPT-15 gene.

2. The yeast strain of claim 1, wherein the mutations at position I46, D56, S118, Y195, and L205 are I46M, D56G, S118P, Y195H, and L205S respectively.

3. The yeast strain of claim 1, wherein the mutated SPT-15 gene encodes an amino acid sequence according to SEQ ID NO 01.

4. The yeast strain of claim 1, wherein the mutated SPT-15 gene comprises nucleic acid sequence according to SEQ ID NO 02.

5. The yeast strain of any of the preceding claims, wherein the at least one copy of the mutated SPT-15 gene is inserted into the genomic region selected from the group of intergenic regions, regulatory regions, genic regions, or combinations thereof.

6. The yeast strain of any of the preceding claims, wherein the yeast strain is of genus selected from the group of *Saccharomyces spp, Kazachstania spp, Torulaspora spp,* and *Pichia spp.*

7. The yeast strain of claim 5, wherein the yeast strain is of genus S. *cerevisiae* or S. *pastorianus.*

8. The yeast strain of any of the preceding claims, further **characterized in that** it expresses a wild-type SPT-15 gene from its genomic locus at levels comparable with wild-type S. *cerevisiae* strains.

9. The yeast strain of any of the preceding claims, **characterized in that** it is deficient in at least one gene selected from the group of ADH1, ADH2, ADH3, ADH4, ADH5, SFA1, ALD6, PDC2, TPI1 or combinations thereof.

10. The yeast strain of any of any of the proceeding claims, wherein the at least one copy of the mutant SPT-15 gene is inserted into the genomic ADH-1 gene locus in a way that said insertion functionally disrupts the ORF of said ADH-1 gene and co-opts its regulatory regions for its expression.

11. The yeast strain according to any of the preceding claims, wherein the recombinant yeast strain is *Saccharomyces cerevisiae* strain BR 23007 deposited under DSM 34577 on 5^{th} of April 2023 at the Institute DSMZ.

12. The yeast strain according to any of the preceding claims, further **characterized in that** it produces about 10% to about 90% less ethanol compared to a wild-type *Saccharomyces cerevisiae* strain.

13. A low-ethanol producing, recombinant yeast strain according to *Saccharomyces cerevisiae* strain BR 23007 deposited under DSM 34577 on 5^{th} of April 2023 at the Institute DSMZ.

14. The yeast strain of claim 13, further **characterized in that**
• the precursor yeast strain is of genus S. *cerevisiae,*
• the mutant SPT-15 gene comprising nucleic acid sequence according to SEQ ID 2 is inserted into the ORF of the ADH-1 locus of the precursor strain, and
• it produces at least 30% less ethanol compared to a wild-type S. *cerevisiae* yeast strain.

15. The use of a recombinant yeast strain according to any of claims 1 to 14 for fermentation or the preparation of alcohol-reduced foods and beverages.
